(19) Europäisches Patentamt
European Patent Office
Office européen des brevets

(11) Veröffentlichungsnummer: **0 008 735**
**A1**

(12) **EUROPÄISCHE PATENTANMELDUNG**

(21) Anmeldenummer: 79103048.9

(22) Anmeldetag: 20.08.79

(51) Int. Cl.³: C 07 C 65/24
C 07 C 51/16

(30) Priorität: 01.09.78 DE 2838269
14.04.79 DE 2915215

(43) Veröffentlichungstag der Anmeldung:
19.03.80 Patentblatt 80/6

(84) Benannte Vertragsstaaten:
BE CH DE FR GB IT NL

(71) Anmelder: BAYER Aktiengesellschaft
Zentralbereich Patente,Marken und Lizenzen Bayerwerk
D-5090 Leverkusen 1(DE)

(72) Erfinder: Horstmann, Walter, Dr.
Eichenweg 17
D-5060 Bergisch Gladbach 2(DE)

(72) Erfinder: Hammerström, Knut, Dr.
Pehlengarten 3
D-5060 Bergisch Gladbach 1(DE)

(54) **Verfahren zur Herstellung von Diphenylether-carbonsäuren.**

(57) Die Erfindung betrifft ein Verfahren zur Herstellung von Diphenylether-carbonsäuren durch Oxidation der entsprechenden Methyldiphenylether im Temperaturbereich von 50 bis 350°C und einem Sauerstoffpartialdruck von 0,1 bis 100 atm, bei dem man methylierte Diphenylether mit einer wäßrigen Alkalidichromatlösung im Temperaturbereich von 150 bis 350°C und bei Drucken bis zu 100 atm oxidiert.

EP 0 008 735 A1

BAYER AKTIENGESELLSCHAFT
Zentralbereich
Patente, Marken und Lizenzen

5090 Leverkusen, Bayerwerk
Mn/bc/Kü/kl

## Verfahren zur Herstellung von Diphenylether-carbonsäuren

Die Erfindung betrifft ein Verfahren zur Herstellung von Diphenylether-carbonsäuren durch Oxidation der entsprechenden Methyldiphenylether.

Aus der DE-OS 2 604 474 ist bekannt, 3-Methyldiphenylether mit Sauerstoff im Bereich von 50 bis 350°C und einem Sauerstoffpartialdruck von 0,1 bis 10 atm in der flüssigen Phase, vorzugsweise einer niederen $C_2$-$C_8$-Alkancarbonsäure als Lösungsmittel, gegebenenfalls in Gegenwart eines Schwermetallkatalysators, zu oxidieren.

Es wurde ein Verfahren zur Herstellung von Diphenylether-carbonsäuren durch Oxidation der entsprechenden methylierten Methyldiphenylether im Temperaturbereich von 50 bis 350°C und bei einem Sauerstoffpartialdruck von 0,1 bis 100 atm gefunden, das dadurch gekennzeichnet ist, daß

man methylierte Diphenylether der Formel

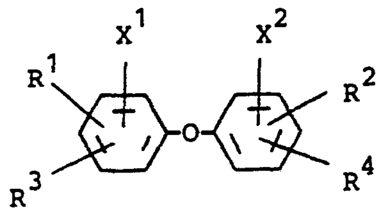

(I)

worin

$X^1$ und $X^2$ gleich oder verschieden sind und für Wasserstoff oder Halogen stehen,

$R^1$, $R^2$ und $R^3$ gleich oder verschieden sind und für Wasserstoff oder Methyl stehen und

$R^4$ Methyl bedeutet,

mit einer wäßrigen Alkalidichromatlösung im Temperaturbereich von 150 bis 350°C und bei Drucken bis zu 100 atm oxidiert.

Das erfindungsgemäße Verfahren kann anhand der folgenden Reaktionsgleichung erläutert werden:

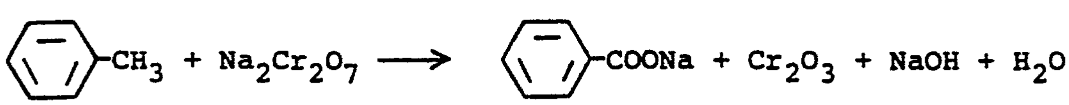

Nach dem erfindungsgemäßen Verfahren können methylierte, gegebenenfalls auch halogenierte, Diphenylether als Einzelverbindungen oder im Gemisch, auch Isomerengemisch, zu den entsprechenden Carbonsäuren oxidiert werden.

Halogen für das erfindungsgemäße Verfahren kann Fluor, Chlor, Brom oder Jod, bevorzugt Fluor, Chlor oder Brom, sein.

Als methylierte, gegebenenfalls auch halogenierte, Diphenylether für das erfindungsgemäße Verfahren seien beispielsweise die folgenden Verbindungen genannt: 2-Methyl-diphenylether, 3-Methyl-diphenylether, 4-Methyl-diphenylether, 3,3'-Dimethyl-diphenylether, 2,3'-Dimethyl-diphenylether, 4,4'-Dimethyl-diphenylether, 2,4'-Dimethyl-diphenylether, 3,4'-Dimethyl-diphenylether, 2-Chlor-3'-methyl-diphenylether, 3-Chlor-3'-methyl-diphenylether, 4-Chlor-3'-methyl-diphenylether, 4-Chlor-4'-methyl-diphenylether, 3-Methyl-6-chlor-diphenylether, 3-Methyl-6-fluor-diphenylether, 4-Brom-3'-methyl-diphenylether, 3,4-Dimethyl-diphenylether, 3,5-Dimethyl-diphenylether, 3,4-Dimethyl-3'-chlor-diphenylether, 3,5-Dimethyl-4'-brom-diphenylether, 3,3',4-Trimethyl-diphenylether, 3,3',4,4'-Tetramethyl-diphenylether und 3,3',5,5'-Tetramethyl-diphenylether.

Die gegebenenfalls halogenierten Mono-, Di-, Tri-, und Tetra-methyl-diphenylether für das erfindungsgemäße Verfahren sind bekannt; so kann man beispielsweise 3-Methyl-diphenylether durch Kondensation von Phenol und m-Kresol an einen Dehydratisierungskatalysator herstellen (DE-OS 2 604 474) oder m-Kresol mit Chlorbenzol in Gegenwart eines Kupferkatalysators gewinnen (JA 104672). Halogenierte Methyl-diphenylether erhält man entsprechend durch Umsetzung entsprechender Dihalogenverbindungen mit Kresolen bzw. Xylenolen. So erhält man beispielsweise 3-Chlor-3'-methyl-diphenylether aus m-Dichlorbenzol und m-Kresol (JA 38561/67). Isomerengemische von Methyldiphenylethern sind entsprechend durch Einsatz von Kresolgemischen und Phenol an einem Dehydratisierungskatalysator bzw. mit Chlorbenzol unter Kupferkatalyse herstellbar.

Gemische von Dimethyl-diphenylethern erhält man als Nebenprodukte der technischen Kresolsynthese durch alkalische Hydrolyse von Chlortoluol oder Chlortoluolgemischen bei erhöhten Drucken und Temperaturen (Industrial and Engineering Chemistry, Volume 38, 254 (1946)).

2,2'-Dimethyl-diphenylether läßt sich nach dem erfindungsgemäßen Verfahren schwerer oxidieren. Daher kann man aus Gemischen der Mono- und/oder Dimethyl-diphenylether mit Hilfe des erfindungsgemäßen Verfahrens den 2,2'-Dimethyl-diphenylether leicht abtrennen. Aus Isomerengemischen von 2,2'-, 2,3'- und 3,3'-Dimethyl-diphenylether, wie sie beispielsweise bei der alkalischen Hydrolyse von 2-Chlortoluol anfallen (Industrial and Engineering Chemistry, Volume 38, 254 (1946)), kann man nach dem erfindungsgemäßen Verfahren ein leicht trennbares Gemisch aus 2,3'- und 3,3'-Dicarboxy-diphenylether erhalten. Isomerengemische aus Mono- und/oder Dicarbonsäuren des Diphenylethers kann man durch nachfolgende chemische und/oder physikalische Trennoperationen, z.B. Umkristallisation, Destillation von Estergemischen und Verseifung der Ester in die Einzelverbindungen auftrennen.

Das erfindungsgemäße Verfahren kann in kontinuierlicher und diskontinuierlicher Arbeitsweise durchgeführt werden.

Die Oxidation wird im allgemeinen im Temperaturbereich von 150 bis 350°C, vorzugsweise von 220 bis 280°C, ausgeführt. Das erfindungsgemäße Verfahren wird im allgemeinen in einem Autoklaven bei Drucken bis zu 100 atm, insbesondere bei Drucken von 30 bis 60 atm, durchgeführt. Die Reaktionszeiten betragen in der Regel 1 bis 20 Stunden.

Als Alkalidichromate für das erfindungsgemäße Verfahren sind im wesentlichen die Natrium-, Kalium- und/oder Ammoniumdichromate zu nennen. Bevorzugtes Alkalidichromat ist das Natriumdichromat.

Es ist selbstverständlich möglich, die Alkalidichromate in Form der entsprechenden Hydrate einzusetzen.

Die Alkalidichromate werden in wäßriger Lösung oder Suspension eingesetzt. Die Konzentration beträgt im allgemeinen 5 bis 50 Gew.-%, bevorzugt 15 bis 35 Gew.-%.

Das Molverhältnis der Methyl-diphenylether zu dem Alkalidichromat liegt im allgemeinen bei 1:1 bis 10, bevorzugt bei 1:1,2 bis 1,8.

Das erfindungsgemäße Verfahren kann beispielsweise wie folgt durchgeführt werden:

In einem Druckbehälter, beispielsweise einem Autoklaven, wird der gegebenenfalls halogenierte Methyl-diphenylether in einer Lösung des Alkalichromats in Wasser suspendiert.

Unter kräftigem Rühren wird auf Reaktionstemperatur erhitzt; dabei stellt sich in der Regel der Reaktionsdruck ein. Nach Beendigung der Umsetzung trennt man die Chromverbindung ab und fällt die Carbonsäure mit einer Mineralsäure, beispielsweise Chlorwasserstoffsäure, aus.

Nach dem erfindungsgemäßen Verfahren entstehen gegebenenfalls halogenierte Diphenylethercarbonsäuren der Formel

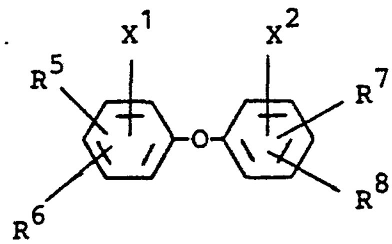

(II)

worin

$X^1$ und $X^2$ die obengenannte Bedeutung haben,

$R^5$, $R^6$ und $R^7$ gleich oder verschieden sind und für Wasserstoff oder die Carboxylgruppe stehen, und

$R^8$ eine Carboxylgruppe bedeutet.

Das erfindungsgemäße Verfahren wird vorteilhafterweise ohne Carbonsäure als Lösungsmittel und ohne Schwermetallkatalysatoren durchgeführt. Die Carbonsäuren werden in hohen Ausbeuten und praktisch ohne Nebenprodukte, wie z.B. Aldehyde, hergestellt.

Diphenylether-carbonsäuren sind Zwischenprodukte bei der Herstellung von Pflanzenschutzmitteln (DE-OS 2 604 474) oder bei der Herstellung von Polymeren (US 2 720 507, GB 839 404, BE 553 897).

Beispiel 1

184 Gew.-Teile 3-Methyl-diphenylether werden in einer Lösung von 450 Teilen Natriumdichromat in 1400 Teilen Wasser suspendiert und in einem 3 l-Edelstahlautoklaven 20 Stunden lang auf 240 bis 250°C erhitzt. Dabei stellt sich ein Druck von 30 bis 40 atm ein. Nach dem Abkühlen saugt man von ausgefallenen Chromverbindungen ab, säuert mit Salzsäure bis zum pH-Wert 1,0 ein, saugt scharf ab und wäscht anteilsweise mit Wasser bis zum farblosen Ablauf nach. Man trocknet im Vakuumschrankt bei 80°C und erhält 208 Teile Diphenylether-3-carbonsäure vom Schmelzpunkt 141 bis 145°C. Nach dem Umlösen aus wäßrigem Alkohol schmilzt das Produkt bei 146 bis 149°C.

Beispiel 2

In zu Beispiel 1 analoger Weise erhält man aus 4-Methyl-diphenylether in 87 %iger Ausbeute die Diphenylether-4-carbonsäure.

Beispiel 3

49,5 Teile 3,3'-Dimethyl-diphenylether. 220 Teile Natriumdichromat und 380 Teile Wasser werden in einem 1,3 l-Edelstahlautoklaven 16 bis 20 Stunden auf 250 ± 5°C erhitzt. Nach dem Abkühlen saugt man ungelöste Chromverbindungen ab und wäscht anteilsweise mit 250 Teilen heißen Wassers aus. Die vereinigten Filtrate säuert man mit halbkonzentrierter Salzsäure bis pH 1,0 bis 1,5 an, saugt den Niederschlag ab und wäscht mit Wasser neutral. Reinigung des Produktes erfolgt in üblicher Weise durch Lösen in verdünnter Natronlauge und Wiederausfällen mit Säure. Nach dem Trocknen erhält man 57 bis 61 Teile Dicarbonsäure vom Schmelzpunkt 252 bis 254°C. Nach einmaligem Umkristallisieren aus Aceton/Wasser (3:1) erhält man 40 Teile analysenreine Substanz vom Schmelzpunkt 253 bis 255°C.

Beispiel 4

148,5 Teile eines technischen Gemisches von Ditolyletherisomeren (Zusammensetzung: 23 % 2,2'-, 21 % 3,3'- und 55 % 2,3'-Dimethyl-diphenylether) werden unter den in Beispiel 1 aufgeführten Reaktionsverbindungen oxidiert. Nach dem Absaugen schwerlöslicher Chromverbindungen und Abtrennen von 45 Teilen unverändertem 2,2'- und 2,3'-Dimethyl-diphenylether wird die wäßrige Phase wie üblich aufgearbeitet. Nach dem Trocknen erhält man 68 Teile Dicarbonsäuregemisch vom Schmelzpunkt 195 bis 203°C; Zusammensetzung: ca. 55 % 2,3'- und 43 % 3,3'-Diphenyletherdicarbonsäure.

Beispiel 5

148,5 Teile eines Ditolyletherisomerengemisches, bestehend aus 20 % 3,3'-, 47 % 3,4'- und 28 % 4,4'-Dimethyl-diphenylether (Rest 2,2'-, 2,3'- und 2,4'-Isomere) werden in einem Edelstahlautoklaven unter den in Beispiel 3 angegebenen Reaktionsbedingungen umgesetzt. Nach dem Abtrennen schwerlöslicher Chromverbindungen und von 28 Teilen unverändertem Ether wird wie im Beispiel 3 beschrieben,

aufgearbeitet und getrocknet. Man erhält 110 Teile eines Dicarbonsäuregemisches vom Schmelzbereich 235 bis 278°C.

Nach Veresterung dieses Gemisches mit Methanol/Schwefelsäure erhält man 105 Teile eines Estergemisches, das aufgrund gaschromatographischer Untersuchungen folgende Zusammensetzung zeigt:

15 % 3,3'-, 61 % 3,4'- und 24 % 4,4'-Diphenyletherdicarbonsäure-dimethylester.

Das Estergemisch schmilzt bei 67 bis 130°C.

Beispiel 6

198 Teile eines Ditolylethergemisches, bestehend aus 3,9 % 3,3'-, 63,2 % 3,4'- und 30,1 % 4,4'-Dimethyldiphenylether (Rest 2,2'-, 2,3'- und 2,4'- Isomere) werden in einer Lösung von 900 Teilen Natrium-dichromat (Dihydrat) in 1360 Teilen Wasser suspendiert und in einem 31-Edelstahlautoklaven 18 Stunden lang auf 240°C erhitzt. Dabei stellt sich ein Druck von 30 - 40 atm ein.

Nach dem Abkühlen auf 60°C saugt man von ungelösten Chromverbindungen ab, wäscht mit 1000 Teilen heißem Wasser anteilsweise nach und säuert die vereinigten Filtrate mit halbkonzentrierter Schwefelsäure bis pH 1-2 an. Das ausgefällte Dicarbonsäuregemisch wird abgesaugt, chromfrei gewaschen und getrocknet. Man erhält 242 Teile eines Produktes, das bei 274 - 280°C schmilzt.

Zur Feststellung der Zusammensetzung des Dicarbonsäuregemisches verestert man eine Probe mit Methanol/Schwefelsäure und untersucht das Estergemisch gaschromatographisch: Dementsprechend enthält das Dicarbonsäuregemisch 3,5 % 3,3'-, 62,7 % 3,4'- und 32,1 % 4,4'- Diphenylether-dicarbonsäure (Rest 2,3'- und 2,4'- Isomere).

Beispiel 7

184 Teile einer Mischung von 73 % 3- und 26 % 4-Phenoxitoluol (Rest m-Kresol) werden in einer Lösung von 450 Teilen Natriumdichromat (Dihydrat) in 1400 Teilen Wasser in einem 3l-Edelstahlautoklaven 20 Stunden lang auf 230-240°C erhitzt. Man arbeitet, wie im Beispiel 3 beschrieben, auf und erhält 201 Teile eines Carbonsäuregemisches vom Schmelzbereich 119-131°C.

Die gaschromatographische Untersuchung einer Probe des analog Beispiel 6 hergestellten Methylestergemisches ergibt für das Carbonsäuregemisch die dem Einsatzprodukt entsprechende Zusammensetzung.

Beispiel 8

198 Teile eines technischen Ditolylethergemisches folgender Zusammensetzung:

| | | |
|---|---|---|
| 6 % | 2,2'- | Ditolylether |
| 26 % | 2,3'- | Ditolylether |
| 12 % | 2,4'- | Ditolylether |
| 25 % | 3,3'- | Ditolylether |
| 24 % | 3,4'- | Ditolylether |
| 6 % | 4,4'- | Ditolylether |
| 1 % | Unbekannte | |

werden, wie in Beispiel 1 beschrieben, oxidiert und aufgearbeitet. Als flüssige organische Phase erhält man 22 Teile Ditolyläthergemisch zurück.

Die Ausbeute an Dicarbonsäuregemisch beträgt 134 Teile vom Schmelzbereich 229 - 253°C. Nach Veresterung der 134 Teile in 360 Teilen Methanol und 50 Teilen Schwefelsäure (100%), Isolieren bei Raumtemperatur, Auswaschen der Mineralsäure und Trocknen erhält man 82 Teile Estergemisch vom Schmelzbereich 88 - 96 °C. Zusammensetzung:

| | | |
|---|---|---|
| 10,1 % | 2,3'- | Diphenylether-dicarbonsäuredimethylester |
| 2,7 % | 2,4'- | Diphenylether-dicarbonsäuredimethylester |
| 44,0 % | 3,3'- | Diphenylether-dicarbonsäuredimethylester |
| 26,6 % | 3,4'- | Diphenylether-dicarbonsäuredimethylester |
| 16,6 % | 4,4'- | Diphenylether-dicarbonsäuredimethylester |

Beispiel 9

218,5 Teile 3-Chlor-3'-methyl-diphenylether werden in einem 3 l Stahl-Autoklaven mit einer Lösung von 450 Teilen $Na_2Cr_2O_7 \cdot 2H_2O$ in 1700 Teilen Wasser 18 Stunden lang bei 230°C oxidiert. Nach dem Abkühlen und Abtrennen des ausgefallenen Chromoxids wäscht man mit 500 Teilen heißem Wasser nach, extrahiert das gekühlte Filtrat zweimal mit je 100 Teilen Toluol, um nicht umgesetztes Ausgangsmaterial zu entfernen, und säuert die wäßrige Phase mit halbkonzentrierter Schwefelsäure an. Man saugt die ausgefällte Chlorcarbonsäure ab, wäscht dichromat- und mineralsäurefrei und reinigt durch Wiederauflösen in verdünnter Natronlauge, Klären nach Zusatz von 10 Teilen

Aktivkohle und Wiederausfällen mit Säure. Nach dem Absaugen des Niederschlages, Auswaschen der Säure und Trocknen erhält man 238 Teile 3-Chlor-diphenylether-3'-carbonsäure vom Schmelzpunkt 124 bis 126°C. Nach 2-maligem Umlösen einer Probe schmilzt die Verbindung bei 127 bis 128°C.

Beispiel 10

In einem 3 l Stahl-Rührwerksautoklaven werden 198 Teile 3,3'-Ditolylether mit einer Lösung von 656 Teilen $Na_2Cr_3O_7 \cdot 2H_2O$ in 1300 Teilen Wasser bei 285°C 45 Minuten lang oxidiert. Dann kühlt man auf 80°C ab, pumpt 98 Teile einer halbkonzentrierten Schwefelsäure zu und erhitzt weitere 60 Minuten auf 285°C. Nach dem Abkühlen auf 50°C saugt man von ausgefallenem Chromoxid ab, wäscht mit 500 Teilen siedend heißem Wasser anteilweise nach und säuert das Filtrat mit halbkonzentrierter Schwefelsäure. Man isoliert die ausgefällte Diphenylether-3,3'-dicarbonsäure, wäscht mit warmem Wasser dichromat- und mineralsäurefrei und trocknet bei 80°C im Vakuum. Die Ausbeute beträgt 237 Teile Dicarbonsäure vom Schmelzpunkt 251 bis 253°C.

Beispiel 11

218,5 Teile eines Isomerengemisches, bestehend aus 96 % 3-Methyl-6-chlordiphenylether und 4 % 4-Methyl-6-chlordiphenylether, werden, wie in Beispiel 7 beschrieben, oxidiert. Nach dem Abtrennen des Chromoxids säuert man das Filtrat mit verdünnter Schwefelsäure an, isoliert das ausgefällte Carbonsäureisomerengemisch, wäscht mit Wasser bis zum farblosen Ablauf des Filtrats nach und trocknet im Vakuum bei 60°C bis zur Gewichtskonstanz.
Man erhält 191 Teile vom Schmelzbereich 183-196°C.
Nach zweimaligem Umkristallisieren aus wäßrigem Alkohol erhält man ein Produkt vom Schmelzpunkt 199-203°C.

Patentansprüche

1) Verfahren zur Herstellung von Diphenylether-carbonsäuren durch Oxidation der entsprechenden Methyldiphenylether im Temperaturbereich von 50 bis 350°C und einem Sauerstoffpartialdruck von 0,1 bis 100 atm, dadurch gekennzeichnet, daß man methylierte Diphenylether der Formel

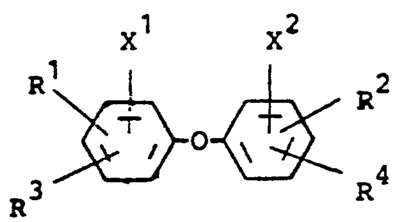

worin
$X^1$ und $X^2$ gleich oder verschieden sind und für Wasserstoff oder Halogen stehen,
$R^1$, $R^2$ und $R^3$ gleich oder verschieden sind und für Wasserstoff oder Methyl stehen und
$R^4$ Methyl bedeutet,

mit einer wäßrigen Alkalidichromatlösung im Temperaturbereich von 150 bis 350°C und bei Drucken bis zu 100 atm oxidiert.

2) Verfahren nach Anspruch 1, dadurch gekennzeichnet, daß man den gegebenenfalls halogenierten Methyldiphenylether und das Alkalidichromat im Molverhältnis von 1:1 bis 10 einsetzt.

3) Verfahren nach den Ansprüchen 1 und 2, dadurch gekennzeichnet, daß man 3-Methyl-diphenylether oxidiert.

4) Verfahren nach den Ansprüchen 1 und 2, dadurch gekennzeichnet, daß man 3,3'-Dimethyl-diphenylether oxidiert.

5) Verfahren nach den Ansprüchen 1 und 2, dadurch gekennzeichnet, daß man Isomerengemische der Monomethyl- oder Dimethyl-diphenylether oxidiert.

6) Verfahren nach den Ansprüchen 1 und 2, dadurch gekennzeichnet, daß man 2-Chlor-5-methyl-diphenylether oxidiert.

7) Verfahren nach den Ansprüchen 1 und 2, dadurch gekennzeichnet, daß man 3-Chlor-3'-methyl-diphenylether oxidiert.

8) Verfahren nach den Ansprüchen 1 und 2, dadurch gekennzeichnet, daß man 2-Fluor-5-methyl-diphenylether oxidiert.

0008735

Europäisches Patentamt

# EUROPÄISCHER RECHERCHENBERICHT

Nummer der Anmeldung

EP 79 103 048.9

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | FR - A1 - 2 256 142 (ISHI ISHARA SANGYO)<br>* Seite 2, Zeile 15 *<br>-- | 1 | C 07 C 65/24<br>C 07 C 51/16 |
| D,A | DE - A1 - 2 604 474 (ICI)<br>* Beispiele 1 und 6, Stufe b *<br>-- | 1,3 | |
| | BEILSTEINS HANDBUCH DER ORGANISCHEN CHEMIE,<br>4. Auflage, 3. Ergänzungswerk, Band 10, 1971, SPRINGER-VERLAG, Berlin, Heidelberg, New York,<br>Seite 289<br>& Chemical Abstracts Band 39, Nr. 10, 20. Mai 1945, Spalte 2246<br>& Journal of the American Chemical Society Band 67, 1945, Seiten 562 bis 564<br>---- | 1,3 | RECHERCHIERTE SACHGEBIETE (Int. Cl.³)<br>C 07 C 51/16<br>C 07 C 65/24 |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 12-12-1979 | KNAACK |